# EUROPEAN PATENT SPECIFICATION

(11) **EP 3 462 860 B1**
(45) Date of publication and mention of the grant of the patent: **16.12.2020**
(21) Application number: 17727187.1
(22) Date of filing: 29.05.2017
(51) Int. Cl.: A01N 1/02, A61B 10/00

(54) **CONTAINER**
BEHÄLTER
CONTENANT

(30) Priority: 01.06.2016 IT UB20164056
(43) Date of publication of application: 10.04.2019
(73) Proprietor: Holostem Terapie Avanzate S.r.l., 41125 Modena (MO) (IT)
(72) Inventor: PELLEGRINI, Graziella, 41125 Modena (MO) (IT); SORANI, Angelo, 41125 Modena (MO) (IT)
(74) Representative: Turri, Elisa
(86) International application number: PCT/EP2017/062891
(87) International publication number: WO 2017/207488

(56) References cited:
- WO-A2-02/072443
- GB-A- 1 038 589
- US-A1- 2003 086 830
- US-A1- 2011 250 106

## Description

### TECHNICAL FIELD

The present invention relates to a container, in particular a container for the transport and/or storage a lentiform and / or substantially circular biological material, preferably like tissue, lamina, flap, or the like. Furthermore, the present invention relates to a kit comprising said container.

### PRIOR ART

Containers for the transport and storage of biological material are known in different forms that are adapted for the particular material to be transported. A characteristic common to all these types of containers is that of being made so as to guarantee a good seal and keep the material to be transported in as sterile an environment as possible. Examples of such containers for the transport of storage of biological material are described in GB-A 1,038,589, US 2011/250106 A1 and WO 02/072443 A2.

Many of these containers have a complex structure determined by the fact that they comprise systems adapted to prevent the deterioration of the biological material transported and in particular to guarantee an absence of toxicity in the short and long term, on the tissue transported. This is the case, for example, of containers for transporting transplant organs that use systems for cooling or regulating the atmosphere internal to the container especially in the case of relatively long transport journeys.

When, instead, the duration of the transport of the biological material is not significant, for example, in the case in which the biological material is used relatively soon after being taken, for example, when the donor and the receiver are the same person, it is not necessary to use sophisticated transport environment control and maintenance systems.

In this case, the container is usually designed to be disposable and is therefore made of plastic material.

However, the use of plastic material is not always optimal for making containers that, for their use, must guarantee a perfect seal, avoid any risk of contamination and however guarantee the absence of toxicity in the short and long term, on the tissue transported.

In fact, the nature of the plastic normally used for containers of this type is not suitable to be smoothed and mirror-polished and therefore a plastic container may have uneven areas in which organic particles could be deposited compromising the sterilization thereof. Furthermore, the closure of a container made of plastic material does not always guarantee a seal against the external environment and can compromise the integrity of the biological material transported.

It is the object of the present invention to overcome the drawbacks mentioned above in relation to known containers and to provide an apparatus that is more effective and functional.

### DESCRIPTION OF THE INVENTION

These objects are reached by a container for the transport and/or storage of organised organic material such as tissue, lamina or the like according to the independent claim 1.

The container according to the present invention comprises a first element having a substantially circular cylindrical shaped cavity for containing said tissue and a circular crown-shaped base coaxial to the cavity, wherein the diameter of the base is greater than the diameter defined by the cylindrical cavity, a second substantially circular shaped element having a horizontal surface and a vertical edge with an inner surface and an outer surface, said second element being couplable to the first element, in such a way that, in a coupling configuration, a closed space is defined between the horizontal surface of the second element and the cavity of the first element. The container further comprises a sealing means positioned between the first and the second element, configured to seal from the outside the space defined by the cavity and the second element.

In particular, the container is characterised in that the first element and the second element are made of mirror-polished steel.

According to the present invention, the first element represents the main body inside which, specifically inside the cavity, the biological material tissue is deposited. The cavity potentially also allows the containment of a liquid inside which the tissue can be immersed. The second element, instead, represents the lid which, when coupled to the first element, hermetically closes the cavity allowing the completely safe transport of the tissue, i.e. preventing any damage or contamination from external agents to the latter. The container is configured so that the closed space defined between the horizontal surface of the second element and the cavity of the first element, adapts perfectly to the shape and size of the tissue material to be transported. In fact, the circular cylindrical shape of the cavity essentially corresponds to the lenticular and/or circular shape of the biological tissue.

This organic material may, for example, be represented by a flap (sheet, or lamina) of corneal epithelium (Holoclar - autologous cells expanded ex vivo from the human corneal epithelium containing stem cells) that looks like a contact lens and that is used for corneal tissue regeneration therapies starting from stem cells, in particular autologous cells.

Since the container according to the present invention is made of mirror-polished steel it can be used various times for its purpose, subject to a suitable sterilisation step.

By using mirror-polished steel, various further advantages are obtained. In fact, the particular smoothing, i.e. the mirror polishing, prevents any uneven areas in which residual organic particles could be deposited and allows more effective sterilisation and cleaning operations. Additionally, the use of steel enables the coupling between the first and second element and therefore the closing of the container itself. Therefore, it is possible to prevent the entry of oxygen and the lowering of the pH, which would jeopardise the vitality of the cells present in the tissue.

In a preferential way, in the coupling configuration, the crown-shaped base and the outer surface of the cylindrical cavity of the first element are in at least partial contact with an inner region of the vertical edge of the second element. In this way, complete adhesion is determined between part of the outer surface of the first element and part of the inner surface of the second element.

In an embodiment of the invention, the sealing means is ring-shaped and the crown-shaped base comprises a circular groove for at least partially receiving said ring. In this way, since in the coupling configuration a region of the vertical edge of the second element is in contact with the crown-shaped base of the first element, pressure is determined between the edge and the base and therefore a hermetic contact zone at the sealing means.

In an alternative embodiment of the invention, the sealing means is ring-shaped and the inner region of the horizontal surface of the second element comprises a circular groove for at least partially receiving said ring. In this way, the hermetic contact zone will not be at the base rather between the horizontal inner surface of the second element and the upper edge of the cylindrical cavity of the first element. In such embodiment, the upper edge of the cylindrical cavity may provide a recess that adapts to the shape of the sealing ring when the second element is coupled to the first element.

In an advantageous way, the coupling between the first and the second element takes place by means of a threaded coupling means arranged in part on the first element and in part on the second element. In this way, the container may be closed or opened simply by screwing or unscrewing the second element with respect to the first element.

Specifically, the threaded coupling means may be provided on the outer surface of the cylindrical cavity of the first element and on the inner surface of the vertical edge of the second element.

Naturally, other coupling means are conceivable. The only condition to be respected is that the second element is to be coupled to the first element hermetically.

In order to improve the action of the sealing means, this means is made of silicone elastomer having a low degree of permanent deformation. A sealing means of this kind has the advantage of being biocompatible, resistant to high temperatures and to chemical agents, sterilisable and adapted to maintain the sterility of the contents inside the container. Furthermore, it is autoclavable and can be made with a "specific shore" suitable for the purpose of a perfect hermetic closure.

According to the present invention, the cylindrical cavity present in the first element, comprises an inner surface and an outer surface. Likewise, the vertical edge of the second element comprises an inner surface and an outer surface.

The diameter of the tissue (or flap) to be transported and/or stored is comprised between 10 mm and 30 mm, preferably between 17 mm and 25 mm and more preferably it has a value of 22 mm.

The cylindrical cavity has an internal diameter between 17.5 mm and 25.5 mm, specifically 23.0 mm. The cylindrical cavity may further have an external diameter, i.e. the outer surface of said cavity, which may be characterised by a threading or other types of closure, may have a diameter comprised between 25 mm and 40 mm, preferably comprised between 34.7 mm and 34.95 mm, specifically M35 X1. In particular, the cavity has a depth comprised between 5 and 15 mm, preferably 10 mm.

In order to prevent any damage to the tissue or flap during transport, the size of the container must be such as to be adapted to the size of the transported material. In particular, the ratio between the internal diameter of the cylindrical cavity and the diameter of the tissue contained therein may be comprised between 1.017 and 1.050, preferably 1.045.

According to an embodiment of the invention, the vertical edge of the second element may have an internal diameter, i.e. the inner surface of said edge characterised by the threading may have a diameter comprised between 25 mm and 40 mm, specifically M35 X1, and an external diameter, i.e. the outer surface of said edge may have a diameter comprised between 30.5 mm and 50 mm, specifically 49.0 mm. In particular, the edge may have a height comprised between 5 mm and 20 mm, in particular 10 mm.

In order to obtain a perfect hermetic closure between the first and the second element, the internal diameter of the vertical edge of the second element coincides with the external diameter of the circular cavity of the first element and assumes a value of M35 X1.

To increase the compactness of the container in the coupling configuration between the first and the second element, the diameter of the crown-shaped base of the first element may correspond to the external diameter of the vertical edge of the second element.

Advantageously, the mirror-polished steel guarantees the absence of the release of metal particles, total biocompatibility, the absence of absorption of biological particles produced by the cells, the absence of biological effects on the stem cells both in the short and long term and the maintenance of the internal sterility of the product contained. Further, it allows the electrochemical etching of permanent data or information on the outside.

According to the present invention, the container can preferably be used for the transport and/or storage of a flap of corneal epithelium used for corneal epithelial regeneration therapy.

The container allows the relative physiological pH and the concentration of carbon dioxide and oxygen to be maintained inside it, for a sufficient amount of time to guarantee the vitality of the flap until the time of the implant. Specifically, in the container there is a proportional quantity of liquid to the quantity of oxygen necessary for the vitality of the flap. Furthermore, the container guarantees the so-called "in-use-stability" in the operating theatre, i.e. the concentration of carbon dioxide dissolved in the volume contained in the container is such as to maintain the vitality of the flap in the operating theatre for a sufficient amount of time, also with the container open at the envisaged times.

The kit according to the present invention comprises a system composed of various containers that can be fitted inside one another. In particular, the kit comprises the container for the transport and/or storage of a lentiform and / or substantially circular tissue of biological material, as described above, wherein said container is fitted into a second container such as, for example, a Petri dish or the like. The size of the second container is such as to guarantee that the container according to the present invention fits perfectly inside it. The kit may further envisage a third container for containing the second container in turn. The third container is represented for example by a self-sealing plastic envelope or the like. The size of the third container must be such as to guarantee that the first and the second containers fit perfectly inside it. In other words, the kit may comprise the container according to the present invention fitted into a second container which is, in turn, fitted into a third container. In an embodiment of the kit, this may comprise a fourth container inside which the three containers mentioned above can be fitted, contained inside one another as described above. A kit of this kind is essentially used for protecting the container for the transport and/or storage of a tissue of biological material according to the present invention and specifically for maintaining it in a sterile environment (prior to its use) during any transport step (for example shipment) or storage. These and other aspects of the present invention will become more apparent in light of the following description of some preferred embodiments described herein below.
Fig. 1 shows a schematic representation of the container according to an embodiment of the present invention;
Fig. 2(a)-(c) show the first element of the container according to the present invention in a lateral and sectional view (a)-(b) and a view from above (c); and
Fig. 3(a)-(b) show the second element of the container according to the present invention in a view from below (a) and a sectional view (b).

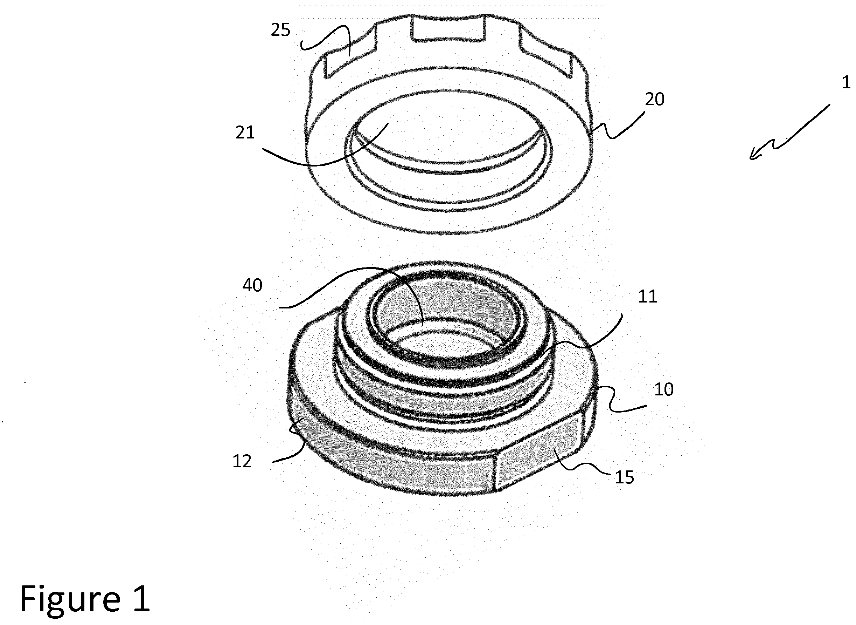

Figure 1 is a schematic representation of the container 1 according to the present invention, in an uncoupled configuration. The container 1 comprises a first element 10, or main body, and a second element 20, or lid, both having a circular shape. Between the first 10 and the second 20 element a sealing means 30 is inserted being ring-shaped and made of silicone (not shown in the figure).

From the figure it can be seen that the first element 10 comprises a cylindrical cavity 11 and a crown-shaped base 12, wherein the diameter of the cylindrical cavity 11 is smaller than that of the base 12. The sealing means 30 may be positioned on the upper surface of the base 12 or within the second element 20 on the horizontal upper surface 21 of the lid. In this way, by coupling the first element 10 with the second element 20, for example, by screwing the lid 20 onto the main body 10, a hermetic contact zone is determined between the two elements at the sealing means 30. It can also be noted from the figure that the second element 20 is equipped with a plurality of specific grooves 25 distributed at an equal distance from one another on the outer surface so as to facilitate the grip of the second element 20 and the consequent screwing or unscrewing in relation to the first element 10. In a similar way, to facilitate the grip of the first element 10, the circular base 12 comprises two flat surfaces 15 opposite one another.

When the first element 10 is coupled with the second element 20, a closed space 40 is formed between the horizontal surface 21 of the second element 20 and the cavity 11 of the first element 10.

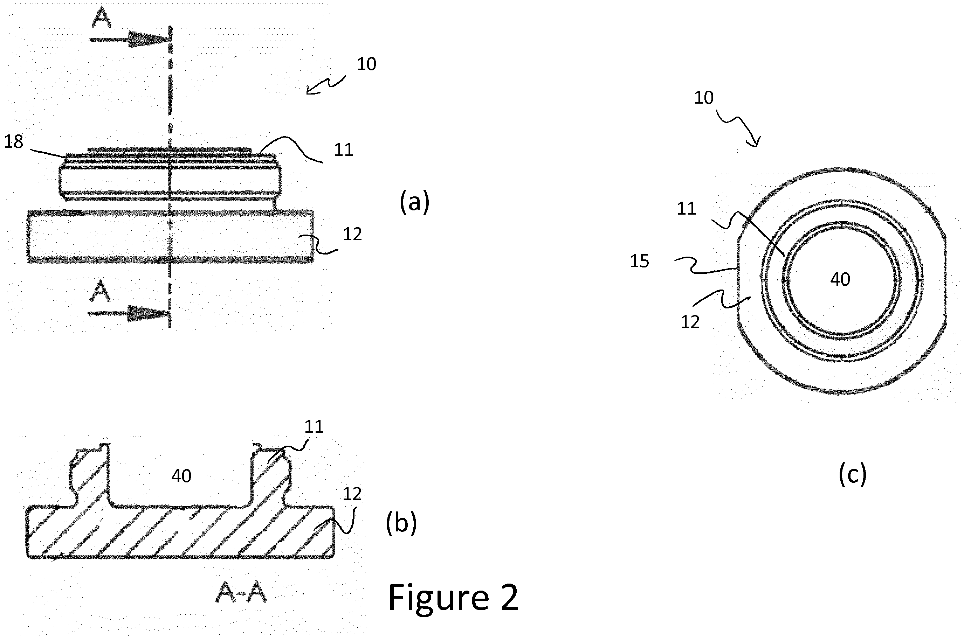

Figure 2a shows the first element 10 in a lateral view. From the figure it can be seen how the cavity 11, with a substantially circular cylindrical shape, is coaxial with the circular crown-shaped base 12, wherein the diameter of the base 12 is greater than the diameter defined by the cylindrical cavity 11.

The first element 10 comprises on the outer surface of the cylindrical cavity 11 a threaded coupling means 18. From the figure, it can be seen how this means 18 is not arranged in a continuous way along the whole surface but is mainly concentrated in some parts of said surface, for example in the upper part.

Figure 2b describes a longitudinal section of the first element 10 shown in figure 2a along the line of section A-A. The cylindrical cavity 11 defines inside it the space 40 which is the space for containing the tissue to be transported once the first element 10 is coupled with the second element 20.

Figure 2c shows the first element 10 in a view from above. From this figure the circular symmetries between the cylindrical cavity 11 and the circular crown-shaped base 12 are even more clear.

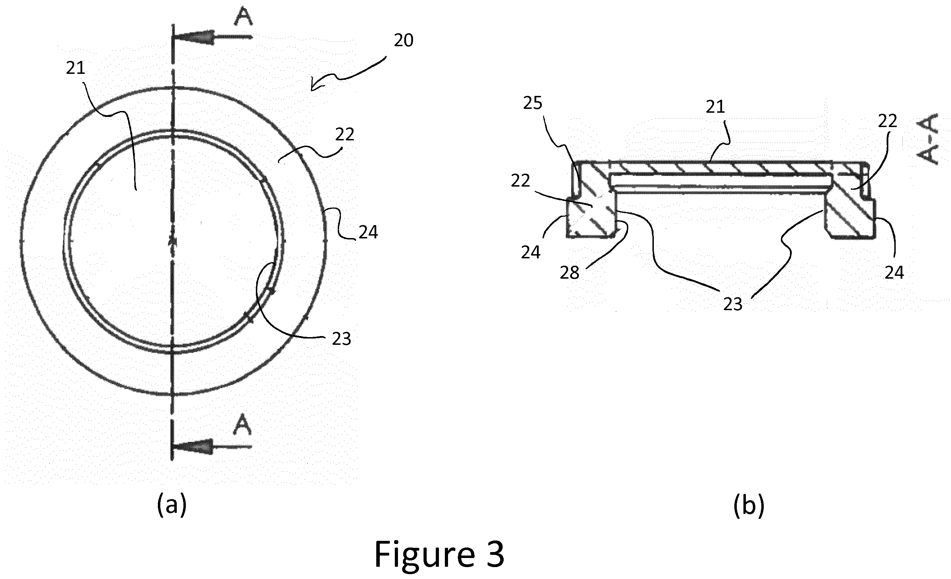

Figures 3a and 3b show the second element 20 in a view from below and in a longitudinal section along the line of section A-A. From the figures it can be seen how the second element 20 comprises a horizontal surface 21 and a vertical edge 22 with an inner surface 23 and an outer surface 24.

As highlighted previously, when the first element 10 is coupled with the second element 20, a closed space 40 is formed between the horizontal surface 21 of the second element 20 and the cavity 11 of the first element 10. The coupling takes place through screwing the first element 10 with respect to the second element 20 thanks to the presence of a threaded means 18 on the outer edge of the cylindrical cavity 11 and a threaded means 28 on the inner surface 23 of the second element 20.

A person skilled in the art can introduce numerous further modifications and variations to the container described hereinabove for the purpose of meeting additional and contingent needs, all of which, however, remaining within the scope of protection of the present invention as defined by the claims attached hereto.

## Claims

1. A container (1) for the transport and / or storage of a lentiform and / or substantially circular biological material, comprising:
a first element (10) having a substantially circular cylindrical shaped cavity (11) for containing said material and a circular crown-shaped base (12) coaxial to the cavity (11), wherein the diameter of the base (12) is greater than the diameter defined by the cylindrical cavity (11);
a second substantially circular shaped element (20) having a horizontal surface (21) and a vertical edge (22) with an inner surface (23) and an outer surface (24), said second element (20) being couplable to the first element (10), in such a way that, in a coupling configuration, a closed space (40) is defined between the horizontal surface (21) of the second element (20) and the cavity (11) of the first element (10), and
a sealing means (30) positioned between the first and the second element (10, 20), configured to seal from outside the space (40) defined by the cavity (11) and the horizontal surface (21),
**characterized in that**
the first element (10) and the second element (20) are made of a mirror-polished steel
and the cylindrical cavity (11) has an internal diameter comprised between 17.5 mm and 25.5 mm and a depth comprised between 5 mm and 15 mm.

2. The container (1) according to claim 1, wherein in the coupled configuration, the crown-shaped base (12) and the outer surface of the cylindrical cavity (11) of the first element (10) are in at least partial contact with an inner region of the vertical edge (22) of the second element (20).

3. The container (1) according to one of the preceding claims, wherein the sealing means (30) is ring-shaped and the crown-shaped base (12) comprises a circular groove for at least partially receiving said ring.

4. The container (1) according to one of claims 1 to 2, wherein the sealing means (30) is ring-shaped and the inner region of the horizontal surface (21) of the second element (20) comprises a circular groove for at least partially receiving said ring.

5. The container (1) according to one of the preceding claims, wherein the coupling between the first and the second element (10, 20) takes place by means of threaded coupling means (18, 28) arranged in part on the first element (10) and in part on the second element (20).

6. The container (1) according to claim 5, wherein the threaded coupling means are arranged on the outer surface of the cylindrical cavity (11) of the first element (10) and on the inner surface (23) of the vertical edge (22) of the second element (20).

7. The container (1) according to one of the preceding claims, wherein the sealing means (30) is made of silicone elastomer having a low degree of permanent deformation.

8. The container (1) according to one of the preceding claims, wherein the ratio between the internal diameter of the cylindrical cavity (11) and the diameter of the material contained therein is comprised between 1.017 and 1.050.

9. The container (1) according to one of the preceding claims, wherein the vertical edge (22) of the second element (20) has an internal diameter comprised between 25 mm and 40 mm, an external diameter comprised between 30 mm and 50 mm and a height comprised between 5 and 20 mm.

10. The container (1) according to one of the preceding claims, wherein the lentiform and / or substantially circular biological material is made at least of a flap of corneal tissue.

11. A kit for the transport and / or storage of a biological material, comprising:
the container (1) according to one of the preceding claims,
a second container such as a Petri dish, for containing the container (1), and
a third container, such as a sealing envelope, for containing the second container.

12. The kit according to claim 11, wherein the lentiform and / or substantially circular biological material is made at least of a flap of corneal epithelium.

13. Use of the container (1) according to one of claims 1 to 10 or of the kit according to claims 11 or 12 for transporting and / or storing a flap of corneal epithelium used for corneal epithelial regeneration therapy.

## Patentansprüche

1. Behälter (1) für den Transport und/oder die Aufbewahrung eines linsenförmigen und/oder im Wesentlichen kreisförmigen biologischen Materials, umfassend:
ein erstes Element (10) mit einem im Wesentlichen kreisförmigen, zylinderförmigen Hohlraum (11) zum Aufbewahren des Materials und einer kreisförmigen, kronenförmigen Basis (12), die koaxial zum Hohlraum (11) ist, wobei der Durchmesser der Basis (12) größer als der durch den zylindrischen Hohlraum (11) definierte Durchmesser ist;
ein zweites im Wesentlichen kreisförmiges Element (20) mit einer horizontalen Oberfläche (21) und einer vertikalen Kante (22) mit einer Innenoberfläche (23) und einer Außenoberfläche (24), wobei das zweite Element (20) mit dem ersten Element (10) derart verbunden werden kann, dass in einer Verbindungskonfiguration ein geschlossener Raum (40) zwischen der horizontalen Oberfläche (21) des zweiten Elements (20) und dem Hohlraum (11) des ersten Elements (10) definiert wird, und
ein Dichtungsmittel (30), das zwischen dem ersten und dem zweiten Element (10, 20) positioniert und konfiguriert ist, um den durch den Hohlraum (11) und die horizontale Oberfläche (21) definierten Raum (40) abzudichten,
**dadurch gekennzeichnet, dass**
das erste Element (10) und das zweite Element (20) aus einem hochglanzpolierten Stahl hergestellt sind und
der zylindrische Hohlraum (11) einen Innendurchmesser zwischen 17,5 mm und 25,5 mm und eine Tiefe zwischen 5 mm und 15 mm aufweist.

2. Behälter (1) nach Anspruch 1, wobei in der verbundenen Konfiguration die kronenförmige Basis (12) und die Außenoberfläche des zylindrischen Hohlraums (11) des ersten Elements (10) mindestens teilweise in Kontakt mit einem Innenbereich der vertikalen Kante (22) des zweiten Elements (20) stehen.

3. Behälter (1) nach einem der vorhergehenden Ansprüche, wobei das Dichtungsmittel (30) ringförmig ist und die kronenförmige Basis (12) eine kreisförmige Nut für das mindestens teilweise Aufnehmen des Rings umfasst.

4. Behälter (1) nach einem der Ansprüche 1 bis 2, wobei das Dichtungsmittel (30) ringförmig ist und der Innenbereich der horizontalen Oberfläche (21) des zweiten Elements (20) eine kreisförmige Nut für das mindestens teilweise Aufnehmen des Rings umfasst.

5. Behälter (1) nach einem der vorhergehenden Ansprüche, wobei die Verbindung zwischen dem ersten und dem zweiten Element (10, 20) mittels Gewindeverbindungsmittel (18, 28), die teilweise auf dem ersten Element (10) und teilweise auf dem zweiten Element (20) angeordnet sind, erfolgt.

6. Behälter (1) nach Anspruch 5, wobei die Gewindeverbindungsmittel an der Außenoberfläche des zylindrischen Hohlraums (11) des ersten Elements (10) und an der Innenoberfläche (23) der vertikalen Kante (22) des zweiten Elements (20) angeordnet sind.

7. Behälter (1) nach einem der vorhergehenden Ansprüche, wobei das Dichtungsmittel (30) aus Silikon-Elastomer mit einem niedrigen Grad an dauerhafter Verformung hergestellt ist.

8. Behälter (1) nach einem der vorhergehenden Ansprüche, wobei das Verhältnis zwischen dem Innendurchmesser des zylindrischen Hohlraums (11) und dem Durchmesser des darin enthaltenden Materials zwischen 1,017 und 1,050 beträgt.

9. Behälter (1) nach einem der vorhergehenden Ansprüche, wobei die vertikale Kante (22) des zweiten Elements (20) einen Innendurchmesser zwischen 25 mm und 40 mm, einen Außendurchmesser zwischen 30 mm und 50 mm und eine Höhe zwischen 5 und 20 mm aufweist.

10. Behälter (1) nach einem der vorhergehenden Ansprüche, wobei das linsenförmige und/oder im Wesentlichen kreisförmige biologische Material aus mindestens einem Hornhautgewebelappen besteht.

11. Kit für den Transport und/oder die Aufbewahrung eines biologischen Materials, umfassend:
den Behälter (1) nach einem der vorhergehenden Ansprüche,
einen zweiten Behälter, wie etwa eine Petrischale, zum Aufbewahren des Behälters (1), und
einen dritten Behälter, wie etwa eine Versiegelungshülle, zum Aufbewahren des zweiten Behälters.

12. Kit nach Anspruch 11, wobei das linsenförmige und/oder im Wesentlichen kreisförmige biologische Material aus mindestens einem Hornhautepithellappen besteht.

13. Verwendung des Behälters (1) nach einem der Ansprüche 1 bis 10 oder des Kits nach Anspruch 11 oder 12 zum Transportieren und/oder Aufbewahren eines Hornhautepithellappens, der zur Regenerationstherapie des Hornhautepithels verwendet wird.

## Revendications

1. Récipient (1) pour le transport et/ou le stockage d'une matière biologique lenticulaire et/ou substantiellement circulaire, comprenant :
un premier élément (10) comportant une cavité cylindrique (11) substantiellement circulaire, destinée à contenir ladite matière, et une base en forme de couronne circulaire (12), coaxiale à la cavité (11), le diamètre de la base (12) étant supérieur au diamètre défini par la cavité cylindrique (11) ;
un deuxième élément substantiellement circulaire (20), présentant une surface horizontale (21) et un bord vertical (22) avec une surface intérieure (23) et une surface extérieure (24), ledit deuxième élément (20) pouvant être accouplé au premier élément (10), de telle façon que dans une configuration d'accouplement, un espace clos (40) est défini entre la surface horizontale (21) du deuxième élément (20) et la cavité (11) du premier élément (10), et
un moyen de scellement (30) positionné entre les premier et deuxième éléments (10, 20), configuré pour sceller depuis l'extérieur l'espace (40) défini par la cavité (11) et la surface horizontale (21),
**caractérisé en ce que**
le premier élément (10) et le deuxième élément (20) sont constitués d'acier poli miroir et
la cavité cylindrique (11) présente un diamètre intérieur compris entre 17,5 mm et 25,5 mm et une profondeur comprise entre 5 mm et 15 mm.

2. Récipient (1) selon la revendication 1, dans lequel, dans la configuration d'accouplement, la base en forme de couronne (12) et la surface extérieure de la cavité cylindrique (11) du premier élément (10) sont au moins partiellement en contact avec la région intérieure du bord vertical (22) du deuxième élément (20).

3. Récipient (1) selon l'une des revendications précédentes, dans lequel le moyen de scellement (30) présente une forme d'anneau et la base en forme de couronne (12) comprend une rainure circulaire destinée à recevoir au moins partiellement ledit anneau.

4. Récipient (1) selon l'une des revendications 1 à 2, dans lequel le moyen de scellement (30) présente une forme d'anneau et la région intérieure de la surface horizontale (21) du deuxième élément (20) comprend une rainure circulaire destinée à recevoir au moins partiellement ledit anneau.

5. Récipient (1) selon l'une des revendications précédentes, dans lequel l'accouplement entre les premier et deuxième éléments (10, 20) est réalisé à l'aide de moyens d'accouplement filetés (18, 28) disposés en partie sur le premier élément (10) et en partie sur le deuxième élément (20) .

6. Récipient (1) selon la revendication 5, dans lequel les moyens d'accouplement filetés sont disposés sur la surface extérieure de la cavité cylindrique (11) du premier élément (10) et sur la surface intérieure (23) du bord vertical (22) du deuxième élément (20).

7. Récipient (1) selon l'une des revendications précédentes, dans lequel le moyen de scellement (30) est constitué d'un élastomère de silicone présentant un faible degré de déformation permanente.

8. Récipient (1) selon l'une quelconque des revendications précédentes, dans lequel le rapport entre le diamètre intérieur de la cavité cylindrique (11) et le diamètre de la matière contenue dans celle-ci est compris entre 1,017 et 1,050.

9. Récipient (1) selon l'une des revendications précédentes, dans lequel le bord vertical (22) de la deuxième élément (20) présente un diamètre intérieur compris entre 25 mm et 40 mm, un diamètre extérieur compris entre 30 mm et 50 mm et une hauteur comprise entre 5 et 20 mm.

10. Récipient (1) selon l'une des revendications précédentes, dans lequel la matière biologique lentiforme et/ou substantiellement circulaire est constituée d'au moins un lambeau de tissu cornéen.

11. Kit pour le transport et/ou le stockage de matières biologiques, comprenant :
le récipient (1) selon l'une des revendications précédentes,
un deuxième récipient tel qu'une boîte de Pétri, destiné à contenir le récipient (1), et
un troisième récipient, tel qu'une enveloppe de scellement, destiné à contenir le deuxième récipient.

12. Kit selon la revendication 11, dans lequel la matière biologique lentiforme et/ou substantiellement circulaire est constituée d'au moins un lambeau d'épithélium cornéen.

13. Utilisation du récipient (1) selon l'une des revendications 1 à 10 ou du kit selon les revendications 11 ou 12, pour le transport et/ou le stockage d'un lambeau d'épithélium cornéen utilisé en thérapie de régénération épithéliale cornéenne.
